(19)

Europäisches
Patentamt
European
Patent Office
Office européen
des brevets

(11) **EP 4 269 382 A1**

(12) **EUROPEAN PATENT APPLICATION**
published in accordance with Art. 153(4) EPC

(43) Date of publication:
**01.11.2023 Bulletin 2023/44**

(21) Application number: **21914750.1**

(22) Date of filing: **23.12.2021**

(51) International Patent Classification (IPC):
**C07C 49/08** (2006.01)  **C07C 45/49** (2006.01)

(86) International application number:
**PCT/ES2021/070931**

(87) International publication number:
**WO 2022/144480 (07.07.2022 Gazette 2022/27)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.12.2020 ES 202031307**

(71) Applicants:
• **Consejo Superior De Investigaciones Científicas
(CSIC)
28006 Madrid (ES)**

• **Universitat Politècnica de València
46022 Valencia (ES)**

(72) Inventors:
• **PRIETO GONZÁLEZ, Gonzalo
46022 Valencia (ES)**
• **ANDRES MARCOS, Eva
46022 Valencia (ES)**
• **GARCÍA FARPON, Marcos
46022 Valencia (ES)**

(74) Representative: **Pons
Glorieta Rubén Darío 4
28010 Madrid (ES)**

(54) **ACETONE PRODUCTION PROCESS**

(57) The present invention relates to a process for the direct synthesis of acetone from synthesis gas and a solid multicomponent catalyst; wherein said multicomponent catalyst integrates at least one carbonylation active component and one ketonisation active component; wherein said carbonylation component comprises a zeotype material having a network structure comprising 8-membered ring units; wherein said ketonisation component comprises a hydroxide, oxide or any combination thereof selected from the list of yttrium, zirconium, titanium, aluminium, silicon, vanadium, niobium, tantalum, chromium, molybdenum, manganese, zinc, gallium, indium, tin, bismuth, lanthanide elements, or any combination thereof.

EP 4 269 382 A1

**Description**

**TECHNICAL SECTOR**

The present invention relates to an acetone production process by bringing a gas mixture that comprises at least synthesis gas into contact with a multicomponent catalyst.

**BACKGROUND**

Acetone, also known as 2-propanone, is an important solvent and chemical intermediate in the synthesis of acrylates and bisphenol-A for the production of polycarbonates and epoxy resins.

The acetone production at an industrial level comprises, in general, a first step consisting of the alkylation of benzene with propylene to produce cumene, which is consecutively oxidised in air to form cumene hydroperoxide. Next, in another reaction step, cumene hydroperoxide is hydrolysed in an acid medium to produce equimolar amounts of phenol and acetone. As a result, the economic profitability of the acetone production process by the method set out is directly related, in turn, to the market demand for phenol. Alternatively, acetone can be obtained through the partial oxidation process of propylene.

The development of acetone production processes from alternative carbon sources is of great interest, such that obtaining it is disassociated from the availability of sources with a high demand, such as alkenes (like propylene) or aromatic hydrocarbons (like benzene), as well as from the market demand for secondary products such as phenol.

Synthesis gas and its direct derivatives constitute an interesting alternative as a carbon source for acetone production. Synthesis gas, commonly known as "syngas", is a mixture typically made up of carbon monoxide (CO), hydrogen $(H)_2$ and, in some cases, also carbon dioxide $(CO_2)$ as main components, which can be obtained from a large number of carbonaceous sources, for example, by steam reforming or partial oxidation of natural gas, through coal gasification, biomass gasification and/or reforming, and carbon dioxide hydrogenation, among others.

Methanol can be obtained directly from synthesis gas through the catalytic process known as methanol synthesis, which consists of the selective hydrogenation of carbon oxides present in the synthesis gas using hydrogen. In the document by J. P. Lange, "Methanol synthesis: a short review of technology improvements", Catalysis Today, volume 64, Issues 1-2, 2001, pages 3-8, an overview of existing catalysts and processes for the direct production of methanol from synthesis gas is presented.

Dimethyl ether (DME) is the ether compound derived from methanol. The production of dimethyl ether from methanol can be carried out in a catalytic dehydration step. In the document by H. Bateni & C. Able, "Development of Heterogeneous Catalysts for Dehydration of Methanol to Dimethyl Ether: A Review", in Catalysis in Industry, volume 11, 2019, pages 7-33, a list of existing catalysts and processes for the conversion of methanol to produce dimethyl ether is described. DME can be produced, alternatively, directly from synthesis gas in a single step by combining the steps of methanol synthesis and its subsequent dehydration. In the document by A. Schäfer et al., US9295978B2 describes a dimethyl ether production process from synthesis gas in a single reaction step. In the document by Q. Fu et al., US8669295B2 describes a process for obtaining a mixture of methanol, dimethyl ether and, in addition, light olefins from synthesis gas in a single step. Therefore, both methanol and dimethyl ether, derived from their dehydration, can be obtained directly from synthesis gas through the processes described in the state of the art.

The development of processes for directly obtaining acetone by converting carbon sources with high availability, such as synthesis gas, or mixtures of synthesis gas and its derivatives such as methanol, dimethyl ether or a combination thereof, is of great interest.

Document CN102653777B describes a process for producing acetone, along with other alcohol-type products, by bringing synthesis gas into contact with microorganisms that carry out fermentation processes, and therefore act as biological catalysts.

The document by V. Berzin et al., "Selective production of acetone during continuous synthesis gas fermentation by engineered biocatalyst Clostridium sp. MAceT113" Letters in Applied Microbiology, volume 55, 2012, pages 149-154, describes a process for producing acetone by fermentation of a synthesis gas stream in the presence of microorganisms designed for said process.

However, these fermentation routes through microorganisms present low yields and productivities at the mild temperatures required for the stability and functionality of the microorganisms and, in general, the reaction products are obtained in the form of highly diluted aqueous solutions, requiring a significant amount of energy to concentrate them.

Document CN111517929A relates to a method for producing acetone from mixtures of dimethyl ether and/or methanol and carbon monoxide by applying an acidic zeolitic catalyst.

Document CN104193606A discloses a process for preparing acetone from synthesis gas comprising several individual reaction and separation steps.

Document GB2212494A describes a process for the direct synthesis of acetone from synthesis gas. According to this process, the synthesis gas is brought into contact with a molecular catalyst dissolved in an organic solvent, wherein said molecular catalyst is obtained by reacting in the absence of oxygen at least one Group VIII transition metal, an organic base, a ligand capable of forming complexes with the metal or metals and a source of anions.

Given the foregoing, there is interest in developing a process for directly producing acetone, in other words, in a single reaction step, without intermediate separation and/or purification steps, from synthesis gas, or alternatively synthesis gas mixtures and synthesis gas derivatives such as methanol, DME or combinations thereof in a process that only uses a solid catalyst. This type of process presents the technical advantages of its use to directly produce acetone, by converting

(i) a synthesis gas stream, or
(ii) an effluent stream from a previous partial conversion process of synthesis gas into methanol and/or dimethyl ether, by means of methods known in the state of the art, wherein the synthesis gas appears mixed with its derivatives of methanol, dimethyl ether or a combination thereof.

The fact that the catalyst is solid is highly beneficial for the implementation of a continuous chemical process since it simplifies its separation, and, where appropriate, recovery of the mixture of reagents and products in the fluid phase, as well as processes for their rejuvenation or regeneration after deactivation.

Therefore, one of the objectives of the present invention is to provide a process for producing acetone by bringing a gas mixture comprising at least synthesis gas into contact with a solid multicomponent catalyst, in a single reaction step.

It has been found that such a process can be implemented by:

(i) Providing a stream comprising synthesis gas.
(ii) Bringing said feed stream into contact with a solid multicomponent catalyst comprising, at least, one carbonylation component and one ketonisation component.

The term "multicomponent catalyst" as used in the context of the present invention is understood as a catalyst comprising the mixture of at least two different solid components that exhibit different catalytic functionalities when said components are used as separate catalysts.

The term "carbonylation" as used in the context of the present invention is understood as the reaction in which a carboxylic compound is formed from an alcohol-type compound or an ether-type compound, and carbon monoxide. The carbonylation component according to the present invention is the component that catalyses this type of reaction.

The term "ketonisation" as used in the context of the present invention is understood as the reaction in which a ketone-type compound, carbon dioxide and water is produced by condensation of two carboxylic compounds or by self-condensation of a single carboxylic compound. The ketonisation component according to the present invention is the component that catalyses this type of reaction.

**DESCRIPTION**

The present invention relates to an acetone production process comprising, in a first step, bringing a gas stream comprising synthesis gas into contact with a solid multicomponent catalyst in one reaction step, and in a second step, recovering acetone from the effluent stream of said reaction step.

Throughout the description and the claims, the term "comprises" and its variants are not intended to exclude other technical features. For a person knowledgeable in the sector of the invention, other purposes, advantages and features of the invention can be derived partly from the description and partly from the embodiment of the invention.

For the process of this invention, it is considered essential that a gas stream comprising synthesis gas is brought into contact with a multicomponent catalyst that at least integrates catalytic functionalities for carbonylation and ketonisation reactions in a single reaction step, without steps of fractionation, isolation, conditioning and/or purification of intermediate products. By means of the process of the invention, the suitable multicomponent catalyst for achieving the beneficial effect of the invention gives rise to the formation of a product stream in which acetone is one of the main organic products.

Therefore, the present invention relates to a process for the direct synthesis of acetone comprising:

(i) providing a multicomponent catalyst,

(ii) providing a feed stream comprising synthesis gas,

(iii) bringing the catalyst provided in (i) into contact with the feed stream provided in (ii) to prepare acetone, and

(iv) recovering acetone from the mixture of reaction products.

More specifically, the present invention relates to a process for the direct synthesis of acetone comprising:

a) reacting a feed stream comprising, at least, synthesis gas on a solid multicomponent catalyst; wherein said multicomponent catalyst integrates at least one carbonylation active component and one ketonisation active component; wherein said carbonylation component comprises a zeotype material having a network structure comprising 8-membered ring units; wherein said ketonisation component comprises a hydroxide, oxide or any combination thereof selected from the list of yttrium, zirconium, titanium, aluminium, silicon, vanadium, niobium, tantalum, chromium, molybdenum, manganese, zinc, gallium, indium, tin, bismuth, lanthanide elements, or any combination thereof.

b) recovering acetone from the stream of said reaction step.

The oxides or hydroxides comprising the ketonisation component relate to any oxide or hydroxide of the mentioned elements (yttrium, zirconium, titanium, aluminium, silicon, vanadium, niobium, tantalum, chromium, molybdenum, manganese, zinc, gallium, indium, tin, bismuth, lanthanide elements), these elements may be acting with any of their possible valences.

### The multicomponent catalyst

According to the present invention, the multicomponent catalyst comprises at least one active component for the carbonylation reaction and at least one active component for the ketonisation reaction.

The carbonylation component in the multicomponent catalyst comprises at least one zeotype comprising channels formed by 8-membered rings (8 MR) in the network structure. The term "zeotype" as used in the context of the present invention refers to the group of zeolites and related crystalline components having structural networks built from $MO_4$ tetrahedrons that share vertices and that define pores and/or cavities with diameters equal to or less than 2 nanometres ($2.10^{-9}$ metres), where M represents a metal or non-metal-type chemical element, mainly being silicon (Si) in the case of zeolites. Structures initially useful for the zeotype include, accordingly, ABW, AEN, AFR, AFV, APC, APD, ATN, ATT, ATV, AVE, AVL, AWO, AWW, BCT, BIK, BRE, CAS, CDO, CFG, CSV, CZP, DAC, DDR, EAB, EEI, EON, EPI, ESV, ETL, EZT, FER, HEU, IHW, IRN, ITE, ITW, JBW, JNT, JSN, JSW, LEV, MAZ, MFS, MOR, MRT, MTF, NSI, OWE, PAR, PCR, PCS, PSI, PTY, PWW, RRO, RTE, RTH, RWR, SAS, SFO, STI, UEI, UFI, VET, YUG, ZON, ACO, AEI, AFN, AFS, AFT, AFX, AFY, ANA, BOZ, BPH, CGS, CHA, CLO, DFO, DFT, EDI, ERI, ETR, ETV, GIS, GME, GOO, IFU, IFW, IFY, IWW, JOZ, KFI, LIT, LOV, LTA, LTF, LYJ, LTL, MEL, MER, MON, MOZ, MWF, NAB, NAT, NPT, OBW, OFF, OSO, PAU, PHI, POR, PUN, PWN, RHO, RSN, RWY, SAT, SAV, SBE, SBN, SBS, SBT, SFV, SFW, SIV, SOR, SOS, STW, SWY, SYT, SZR, THO, TSC, TUN, UOE, USO, UOV, VNI, VSV, WEI, WEN, YFI-type structures and mixed structures made up of two or more of these structure types.

In a preferred embodiment of the invention, the carbonylation component in the multicomponent catalyst comprises a zeolite. In addition to Si and O, the suitable zeolite structure network may contain at least one trivalent element selected from Al, Ga, B, In, Y, La, Fe or combinations thereof. These elements can be introduced into the zeolite network by any of the conventional methods. In a more preferred embodiment of the invention, the zeolite structure network includes Al, Ga or any combination thereof. In an even more preferred embodiment of the invention, the zeolite structure network includes Al.

In a preferred embodiment of the invention, the zeolite structure network in the carbonylation component of the multicomponent catalyst comprises the trivalent element Al and where the $SiO_2/Al_2O_3$ molar ratio in the zeolite composition is in the range of 3 to 100. Even more preferably, said ratio is in the range of 8 to 40.

In a preferred embodiment of the invention, the carbonylation component in the multicomponent catalyst comprises a zeolite with a MOR, ETL, FER, CHA, SZR-type structure or any combination thereof.

Optionally, the zeolite of the carbonylation component has been modified by incorporating one or more metals such as Ag, Cu, Ir, Pd, Pt, Rh, Co, Re or Zn, preferably Ag, Cu, Pd or any combination thereof. These elements are not incorporated into the network, but rather deposited on the zeolite surface (which includes both the inner surface of its pores and cavities and the outer surface of the crystals). Several methods for incorporating these optional metals into the zeolite component are known in the technical sector. These methods comprise preparation techniques such as impregnation, deposition-precipitation, ion exchange, electrostatic adsorption, co-precipitation, infiltration of molten salts, or solid state deposition. The documents "Handbook of Heterogeneous Catalysis", G. Ertl, H. Knoezinger, F. Schüth, J. Weitkamp (Editors); volume 1, Wiley-VCH Verlag GmbH & Co. Weinheim, 2008 and "Synthesis of solid catalysts", K. P. de Jong (Editor); Wiley-VCH Verlag GmbH & Co. Weinheim, 2009 provide references to existing methods for incorporating metals into an inorganic support material, such as a zeotype.

Regarding the ketonisation component in the multicomponent catalyst, useful components are initially any suitable oxide, hydroxide or combination thereof. Although a large number of metal, non-metal and lanthanide oxides may show activity for ketonisation, in the context of the present invention useful ketonisation components in the multicomponent catalyst must necessarily be stable, that is, they may not be reduced to the metallic state or totally or partially transformed into an inactive carbide-type compound for ketonisation, in the presence of the feed stream comprising synthesis gas under the conditions of the process of the invention. Suitable oxides and hydroxides for the process of the present invention comprise all those selected from the list of compounds of yttrium oxide and hydroxide, zirconium, titanium, aluminium, silicon, vanadium, niobium, tantalum, chromium, molybdenum, manganese, zinc, gallium, indium, tin, bismuth, lanthanide elements, or any combination thereof, in an unsupported mode or combined with a porous material such as those conventionally used as supports for supported catalysts, such as $SiO_2$, $Al_2O_3$, $TiO_2$, $ZrO_2$, zeolites, SiC, carbon or combinations thereof. Preferably, the ketonisation component of the multicomponent catalyst comprises $CeO_2$, $ZrO_2$ or any combination thereof. More preferably, the ketonisation component of the multicomponent catalyst may comprise, in addition to $CeO_2$, $ZrO_2$ and any combination thereof, elements selected from the list of manganese, titanium, other lanthanides, and any combination thereof. More preferably, the ketonisation component of the multicomponent catalyst may comprise, in addition to $CeO_2$, $ZrO_2$ and any combination thereof, a metal selected from those of groups 8-11 of the periodic table. Even more preferably, the ketonisation component of the multicomponent catalyst may comprise, in addition to $CeO_2$, $ZrO_2$ and any combination thereof, the metal Pd.

In a preferred embodiment of the invention, the multicomponent catalyst incorporates, additionally, a suitable hydrogenation component for the hydrogenation of synthesis gas to methanol, DME or combinations thereof. In the context of the present invention, said hydrogenation component comprises at least one oxide selected from ZnO, $ZrO_2$, MgO, $In_2O_3$, $Ga_2O_3$, $CeO_2$ or any combination thereof. In another preferred embodiment of the present invention, said hydrogenation component comprises, in turn, an oxide-supported metal, selected from Cu, Pd or any combination thereof. In a more preferred embodiment of the invention, the hydrogenation component comprises at least one acidic solid selected from $Al_2O_3$, in its forms of γ-alumina, η-alumina, χ-alumina, other transition aluminas, amorphous $SiO_2$-$Al_2O_3$, a zeolite, or combinations thereof.

According to the present invention, the different components are integrated into a multicomponent catalyst. This integration can be achieved by mixing the solid components by different methods, giving rise to different levels of spatial proximity between the catalytically active species thereof.

In a particular embodiment of the present invention, the integration of the components in the multicomponent catalyst comprises the deposition of nanometre or micrometre-sized particles from one of the components onto the surface of the particles of the other component. Several methods for incorporating and dispersing nanometre or micrometre-sized particles from materials onto the surface of solids are known in the technical sector. These methods comprise preparation techniques such as impregnation of suitable precursor salts, deposition-precipitation, co-precipitation, infiltration with molten salts, solid state deposition, or surface-induced crystallisation, optionally followed by heat treatments such as calcination.

In another particular embodiment of the present invention, the components are integrated by solid-state grinding of the individual components in a mortar or mill such that the multicomponent catalyst is formed as a composite material from the individual components in their powder form.

In another embodiment of the present invention, the components are integrated by mixing macroscopic particles or shaped bodies thereof, either in a spatially random or spatially structured manner in a reactor where the process of the present invention will be carried out, such that the multicomponent catalyst comprises a mixture of shaped bodies of the individual components.

The term "shaped bodies" as used in the context of the present invention comprises several particulate and macroscopic forms into which solid catalysts are moulded for application, and which are produced by methods known in the technical sector such as pelleting, extrusion, granulation or spray drying. In addition to the carbonylation and ketonisation components, the multicomponent catalyst may comprise binders and additives that are typically incorporated with the aim of improving the porosity or mechanical properties of such shaped bodies.

In a particular embodiment of the present invention, the multicomponent catalyst is subjected to an activation treatment, prior to the start of the acetone synthesis reaction. In another particular embodiment of the present invention, said activation treatment is carried out in the same reactor where acetone synthesis will be carried out. In a preferred embodiment of the present invention, said activation treatment consists of one or more heat treatment steps at one or more temperatures selected in the range of 373 K to 1073 K, after having established in the reactor a gas flow selected from the list of air, oxygen, nitrogen, helium, argon, hydrogen, carbon monoxide, carbon dioxide, and any combination thereof. In an even more preferred embodiment of the present invention, said activation treatment consists of a first heat treatment step at a temperature selected in the range of 373 K to 1073 K, after having established in the reactor a gas flow selected from the list of air, oxygen, nitrogen, helium, argon, and any combination thereof, followed by a second heat treatment step at a temperature selected in the range of 373 K to 623 K, after having established in the reactor a gas flow comprising at least hydrogen, carbon monoxide or any combination thereof.

*Process conditions*

According to one embodiment of the present invention, the reaction step of the process takes place in a single reactor under a single set of reaction conditions (temperature, pressure and gas-solid contact time). The reagents can be introduced in the gas phase or in the liquid phase, followed by vaporisation of the liquids to form a gaseous feed stream.

The feed stream to the process of the invention comprises synthesis gas. According to the present invention, the $H_2/CO$ molar ratio in synthesis gas can be found in the range of 0.1 to 4. In a preferred embodiment of the present invention, said $H_2/CO$ molar ratio in synthesis gas is in the range of 0.3 to 2.5. More preferably, the $H_2/CO$ molar ratio in synthesis gas is in the range of 0.5 to 2.

According to the present invention, the $CO_2/CO$ molar ratio in synthesis gas can be found in the range of 0 to 2. In a preferred embodiment of the present invention, said $CO_2/CO$ molar ratio in synthesis gas is in the range of 0 to 0.5. More preferably, the $CO_2/CO$ molar ratio in synthesis gas is in the range of 0 to 0.1.

In a particular embodiment of the present invention, the feed stream to the process comprises, in addition to synthesis gas, an organic compound selected from methanol, DME, or a mixture thereof. In a preferred embodiment of the present invention, the CO/(methanol+DME) molar ratio in the feed stream to the process is greater than 10, and more preferably greater than 20.

The feed stream to the process of the present invention may additionally comprise other compounds that do not interfere with the conversion of synthesis gas, methanol and/or DME into acetone, such as nitrogen, helium, argon, water, methane, ethane or propane.

In a preferred embodiment of the present invention, the feed stream to the process originates from a previous reaction step where a synthesis gas stream is partially converted into methanol, DME or any combination thereof.

According to the present invention, the process is carried out at a reaction temperature in the range of 373 degrees Kelvin (K) to 673 K. The reaction temperature as used in the context of the present invention is understood as the maximum temperature in the reactor containing the multicomponent catalyst measured by a type K thermocouple located inside a stainless steel sheath. In a preferred embodiment of the present invention, the process is carried out at a reaction temperature in the range of 473 K to 613 K. More preferably, the process is carried out at a reaction temperature in the range of 493 K to 573 K.

Typical operating pressures suitable for the process of the invention are from approximately 1 bar to approximately 200 bar. In a preferred embodiment of the present invention, the process is carried out at a reaction pressure in the range of 5 bar to 200 bar. More preferably, the process is carried out at a reaction temperature in the range of 10 bar to 150 bar.

The products of the inventive process typically flow from the reactor in the gas phase. In one embodiment of the invention, the reactor effluent stream is fractionated downstream of the reactor, in other words, to recover the acetone product.

In another particular embodiment of the invention, compounds present in the reactor effluent stream, such as reagents present in the feed stream to the process that have not been converted during their passage through the reactor, reaction products, or a combination thereof, are recovered and recirculated to the reactor. In a preferred embodiment of the present invention, a stream comprising carbon monoxide, carbon dioxide, hydrogen, methanol, DME, acetic acid, methyl acetate, or any combination thereof, is recovered from the reactor effluent stream and recirculated to the reactor.

In another particular embodiment of the invention, the mixture of compounds recovered from the reactor effluent stream comprising methanol is subjected to a dehydration step, in another reactor, where methanol is converted, fully or partially, into DME, followed by a step of total or partial removal of water, prior to its recirculation to the reactor of the process of the invention.

**DESCRIPTION OF THE FIGURES**

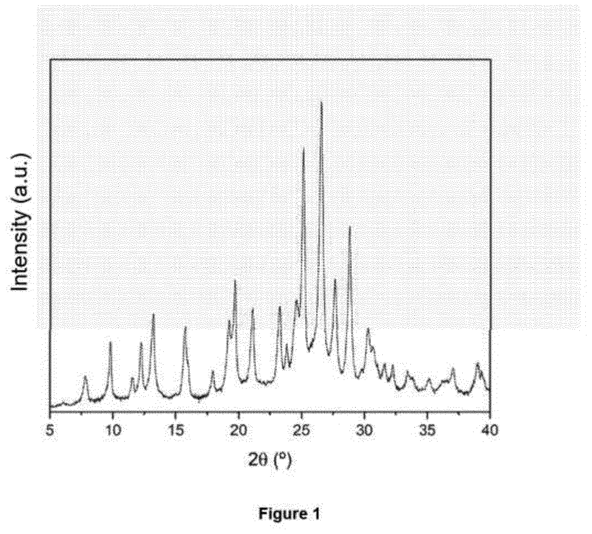

**Figure 1** shows the X-ray powder diffractogram for the H-ETL material according to examples 1, 2, 3, 5 and 12 of the present invention. The X-axis corresponds to the angle (2θ, in degree units), increasing from left to right, while the Y-axis corresponds to the diffraction signal intensity in arbitrary units of relative counts, increasing from bottom to top.

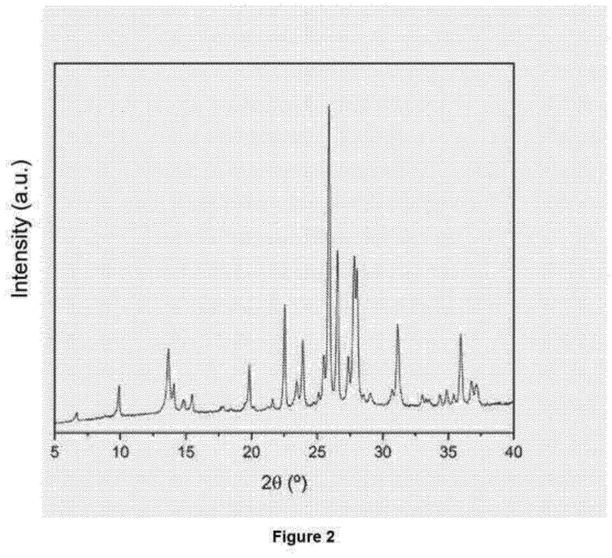

**Figure 2** shows the X-ray powder diffractogram for the Ag-H-MOR material according to examples 4, 6, 7, 8 and 9 of the present invention. The X-axis corresponds to the angle (2θ, in degree units), increasing from left to right, while the Y-axis corresponds to the diffraction signal intensity in arbitrary units of relative counts, increasing from bottom to top.

**EXAMPLES**

The following examples are provided by way of illustration, but are not intended to limit the scope of the present invention.

The following methods have been used to determine the properties of the materials prepared and used in the following examples:

(i) The macroscopic size of the catalyst particles was determined using Retsch calibrated stainless steel sieves.

(ii) The crystalline structure of the materials was determined by means of X-Ray Powder Diffraction. Experimentally, the measurements were taken in Bragg-Brentano geometry using a PANalytical CUBIX diffractometer equipped with a PANalytical X'Celerator detector. X-ray radiation of Cu K$\alpha$ ($\lambda 1$ = 1.5406 Å, $\lambda 2$ = 1.5444 Å, I2/I1 = 0.5) generated in a copper metal anode source operated at a voltage of 45 kV and an intensity of 40 mA was used. The length of the goniometer arm is 200 mm, and a variable divergence slit with an irradiated sample area of 5 mm was used. The measurement range used was from 2.0° to 40.0° (2θ), with a step of 0.020° (2θ) and a measurement time of 35 seconds per step. The measurements were made at 298 K, while the sample, mounted as a fine powder on a sample holder with a sample area of 79 mm$^2$ or 804 mm$^2$, rotated at 0.5 revolutions per second about the axis perpendicular to the surface of the irradiated sample.

iii) The chemical composition of the zeolitic materials comprised in the carbonylation component was determined by inductively coupled plasma-optical emission spectroscopy (ICP-OES), using a Varian 715-ES spectrometer. The samples were previously dissolved in a mixture of nitric acid (HNOs) and hydrochloric acid (HCl), in a 1:3 (HNO$_3$:HCl) ratio, at 333 K for 20 h.

***Synthesis of a multicomponent catalyst according to the present invention***

Synthesis of H-ETL zeolite (SiO$_2$/Al$_2$O$_3$ = 16)

The ETL zeolite was synthesised in its aluminosilicate form (EU-12). First, a synthesis gel was synthesised by mixing 0.4 g of sodium hydroxide, NaOH, (Sigma Aldrich, 97% purity), previously dissolved in deionised water, 4.78 g of rubidium hydroxide solution (RbOH, 50% aqueous solution, Sigma Aldrich, 99.9%), 4.65 g of choline chloride (ChCl, Sigma Aldrich, ≥ 99%), previously dissolved in deionised water, 0.80 g of aluminium hydroxide monohydrate, Al(OH)$_3$.H$_2$O (Sigma Aldrich, reagent-grade purity), 12.5 g of colloidal silica (Sigma Aldrich, LUDOX AS40, 40% suspension in water), and the required amount of deionised water (conductivity < 1 $\mu$S/cm) to achieve a molar composition of the gel of 2.0 ChCl:0.7 Rb$_2$O:0.3 Na$_2$O:0.25 Al$_2$O$_3$:5.0 SiO$_2$:100 H$_2$O. The gel was kept stirring for 24 hours, using a magnetic stirrer, at 250 rpm, at room temperature. Next, the synthesis gel was divided into two stainless steel autoclaves fitted with 35 ml capacity Teflon® sheaths. The autoclaves were introduced into an oven preheated to 423 K and maintained at that temperature, under rotary stirring (60 rpm) for 8 days. Then, the obtained solid was recovered from the rest of the gel by filtration and washed with abundant deionised water. Lastly, the obtained solid was dried in a stove at 373 K and subsequently calcined in a tubular reactor at 823 K (2 K/min) for 4 h, packed in the form of a fixed bed and under synthetic air flow (approximately 80 ml/min). Subsequently, the calcined solid was subjected to three ion exchange steps by suspending it in a 0.5 M NH$_4$NO$_3$ solution in deionised water, mediating the recovery of the solid by filtering after each of the ion exchange steps. After ion exchange, the recovered solid was air-dried at 373 K for 5 hours. Lastly, the solid was subjected to an additional calcination step in a fixed-bed tubular reactor at 823 K (2 K/min) for 4 h and under synthetic air flow (approximately 80 ml/min).

Synthesis of H-MOR zeolite (SiO$_2$/Al$_2$O$_3$ = 18)

For the synthesis of the H-MOR material, 3 g of mordenite zeolite in its ammonium form (NH$_4$-MOR, SiO$_2$/Al$_2$O$_3$ molar ratio provided by the supplier equal to 20, Zeolyst International), were subjected to calcination heat treatment in a fixed-bed tubular reactor under synthetic air flow (approximately 80 ml/min), by heating from 298 K to 823 K (heating rate of 3 K/min) and an isothermal step at 823 K for 4 hours.

Synthesis of modified silver-modified H-MOR zeolite (Aq-H-MOR) (SiO$_2$/Al$_2$O$_3$ = 18)

For the synthesis of the Ag-H-MOR material, 7.65 g of mordenite zeolite in its ammonium form (NH$_4$-MOR, SiO$_2$/Al$_2$O$_3$ molar ratio provided by the supplier equal to 20, Zeolyst International), were suspended in 45 ml of deionised water (conductivity < 1 $\mu$S/cm). 1.00 g of silver nitrate (AgNOs, Sigma Aldrich, ≥ 99%) was dissolved in 4 ml of deionised water. The AgNOs solution was added to the NH$_4$-MOR suspension and kept stirring at room temperature for 30 minutes. The solvent was evaporated under vacuum using a rotary evaporator at 323 K. Then, the obtained material was dried at 373 K for 2 h and subsequently subjected to drying and subsequent calcination in a non-convection muffle oven, in air atmosphere, using a thermal programme at 383 K for 4 hours, followed by a heating step up to 773 K, by means of a temperature ramp of 3 K/min, and a final isothermal step at 773 K for 3 hours.

## Synthesis of modified Ag-H-MOR zeolite (mod-Ag-H-MOR)

2.5 g of the Ag-H-MOR zeolite were shaped into microparticles in the size range of 200 - 400 $\mu$m and mixed with silicon carbide (SiC) microparticles in the size range of 600 - 800 $\mu$m. Subsequently, the mixture of the microparticles was introduced into a fixed-bed tubular reactor on a quartz wool support. The total volume of the fixed bed was 1.5 ml. First, heating was carried out from room temperature to 773 K (with a temperature ramp of 3 K/min) under a downward vertical flow of $N_2$ (Abelló-Linde, 99.999%) at 50 ml/min, followed by an isothermal step at 773 K for 3 hours and cooling to room temperature. Next, a gas stream flow containing DME/CO/$H_2$/Ar in molar ratios of 1/45/45/9 of approximately 80 ml/min was established in the reactor, and the reactor was pressurised up to 20 bar pressure through a diaphragm pressure regulation valve (Swagelok), located downstream of the reactor. Next, the reactor temperature was increased to 548 K following a heating ramp of 3 K/min and, subsequently, it was maintained at a constant temperature of 548 K for 15 hours and subsequently cooled to room temperature. Finally, the zeolite microparticles (mod-Ag-H-MOR) were recovered from the mixture of microparticles from the bed by sieving.

## Synthesis of H-FER zeolite

The H-FER zeolite was synthesised in the form of aluminosilicate. A synthesis gel was prepared by mixing 0.79 g of CATAPAL pseudo-boehmite (Sasol Materials, 72%), 10.63 g of trans-1,4-Diaminocyclohexane (TDACH, Sigma Aldrich, 98%), 25.33 g of colloidal silica (Sigma Aldrich, LUDOX AS40, 40% suspension in water) and deionised water (conductivity < 1 $\mu$S/cm). The mixture was kept stirring while the amount of water required to achieve the molar composition of the gel 1 $SiO_2$: 0.033 $Al_2O_3$ : 0.48 TDACH : 5 $H_2O$, was evaporated using a mechanical stirrer, at 250 rpm, at room temperature. Once the required amount of water has been adjusted to said gel composition, 3.52 g of hydrofluoric acid (Sigma Aldrich, 48% in water) were added to said gel, obtaining a final composition of the gel 1 $SiO_2$: 0.033 $Al_2O_3$ : 0.48 TDACH : 0.5 HF : 5 $H_2O$. The prepared gel was kept stirring for approximately another 30 minutes, at room temperature. Next, the synthesis gel was divided into three stainless steel autoclaves fitted with 35 ml capacity Teflon® sheaths. The autoclaves were introduced in an oven preheated to 423 K and maintained at that temperature for 15 days.
Then, the obtained solid was recovered from the rest of the gel by filtration and washed with abundant deionised water. Lastly, the obtained solid was dried in a stove at 373 K and subsequently calcined in a tubular reactor at 823 K (1 K/min) for 10 h, packed in the form of a fixed bed and under synthetic air flow (approximately 80 ml/min).

## Synthesis of praseodymium-doped cerium oxide (Pr-CeO$_2$)

For the synthesis of the Pr-CeO$_2$ material, 0.56 g of praseodymium nitrate hexahydrate, $Pr(NO_3)_3 \cdot 6H_2O$ (Alfa Aesar, 99.99%), were mixed with 5.09 g of cerium nitrate hexahydrate, $Ce(NO_3)_3 \cdot 6H_2O$ (Alfa Aesar, 99.99%), by vigorous grinding in a ceramic mortar until a uniform mixture of both solids is obtained. Then, the mixture was subjected to calcination in a non-convection muffle oven, in air atmosphere, using a thermal programme in which the temperature was increased at 1 K/min up to a temperature of 673 K, followed by a final isothermal step at 673 K for 5 hours.

## Synthesis of zirconium-doped cerium oxide (CeO$_2$-ZrO$_2$)

The CeO$_2$-ZrO$_2$ material was synthesised by subjecting the mixed oxide of cerium (IV) and zirconium (IV) (Sigma-Aldrich, 99%) to a heat treatment consisting of heating from room temperature to 773 K (with a temperature ramp of 3 K/min) under $N_2$ flow (Abelló-Linde, 99.999%) at approximately 100 ml/min g, followed by an isothermal step at 773 K for 4 hours and cooling to room temperature.

## Synthesis of Pd-modified zirconium-doped cerium oxide (Pd-CeO$_2$-ZrO$_2$)

For the synthesis of the Pd-CeO$_2$-ZrO$_2$ material, 2 g of CeO$_2$-ZrO$_2$, synthesised according to the previously described process, were suspended in 50 ml of acetone. 4.1 mg of palladium acetylacetonate (Pd(acac)$_2$, Sigma Aldrich, 99%) were dissolved in 5 ml acetone. The Pd(acac)$_2$ solution was added to the CeO$_2$-ZrO$_2$ suspension and kept stirring at room temperature for 20 minutes. The solvent was evaporated under vacuum using a rotary evaporator at 303 K. Then, the obtained material was dried at 333 K for 4 h and subsequently subjected to drying and subsequent calcination in a non-convection muffle oven, in air atmosphere, using a thermal programme at 383 K for 4 hours, followed by a heating step up to 873 K, by means of a temperature ramp of 1 K/min, and a final isothermal step at 873 K for 4 hours.

Synthesis of H-FER and Pd-CeO$_2$-ZrO$_2$ composite (H-FER/ Pd-CeO$_2$-ZrO$_2$)

For the preparation of the composite material, 212 mg of H-FER and 1.59 g of Pd-CeO$_2$-ZrO$_2$ were mixed in the form of fine powders with the help of a ceramic mortar until a powdered solid with a uniform appearance was obtained.

Synthesis of aluminium oxide-supported praseodymium oxide, Pr$_2$O$_3$/Al$_2$O$_3$

The Pr$_2$O$_3$/Al$_2$O$_3$ material was synthesised by impregnation. In a first step, 1.90 g of aluminium oxide, produced by calcination of a pseudo-boehmite-type DISPERAL HP14 precursor (Sasol Materials, Germany) at 823 K in a non-convection muffle oven in air atmosphere, were dried in a multi-neck round bottom flask at a temperature of 473 K under dynamic vacuum provided by a vacuubrand-MZ-2C-NT diaphragm pump for 4 hours. 1.84 g of praseodymium nitrate, Pr(NO$_3$)$_3$.6H$_2$O (Alfa Aesar, 99.99%), were dissolved in 2 ml of deionised water. 1.33 ml of the praseodymium nitrate solution were brought into contact with the previously dried aluminium oxide, at room temperature and under static vacuum, allowing the solution to infiltrate the pores of the aluminium oxide support. The obtained material was subjected to a drying treatment in a fixed-bed tubular reactor under synthetic air flow (approximately 80 ml/min) at 343 K for 10 hours, followed by a calcination treatment in the same reactor, and under the same synthetic air flow, by heating from 343 K to 773 K (heating rate of 3 K/min) and an isothermal step at 773 K for 3 hours.

Synthesis of mixed copper, zinc and aluminium oxide (CuO/ZnO/Al$_2$O$_3$)

The CuO/ZnO/Al$_2$O$_3$ material was synthesised by co-precipitation. 3.84 g of copper nitrate, C$_U$(NO$_3$)$_2$.2.5H$_2$O (Alfa Aesar, 98.0-102.0%), 2.67 g of zinc nitrate, Zn(NO$_3$)$_2$.6H$_2$O (Alfa Aesar, 99%), and 1.67 g of aluminium nitrate, Al(NO$_3$)$_3$.9H$_2$O (ACROS, 99%), were dissolved in 30 ml of deionised water. The nitrate solution was added at a constant rate of 2 ml/min, using a syringe pump, on 50 ml of deionised water, which pH had been adjusted to 7 by adding a diluted Na$_2$CO$_3$ solution, maintained at 338 K in an oil bath and under magnetic rotary stirring (350 rpm). pH was kept constant during the incorporation of the nitrate solution by simultaneous adding a Na$_2$CO$_3$ solution (1.5 M). The precipitate formed was kept in its mother liquors at 338 K, with magnetic stirring, for 2 hours. The synthesised solid was recovered by filtration, for which 7 steps of filtering and washing with deionised water were carried out. The obtained precursor was subjected to calcination in a non-convection muffle oven, in air atmosphere, using a thermal programme in which the temperature was increased at 2 K/min up to a temperature of 673 K, followed by a final isothermal step at 673 K for 4 hours.

Synthesis of aluminium oxide ($\gamma$-Al$_2$O$_3$)

The $\gamma$-Al$_2$O$_3$ material was synthesised by calcination of a dispersible pseudo-boehmite-type precursor (DISPERAL HP14 (Sasol Materials, Germany)). 5 g of the pseudo-boehmite precursor were calcined in a non-convention muffle oven, in air atmosphere, using a thermal programme in which the temperature was increased at 2 K/min up to a temperature of 823 K, followed by a final isothermal step at 823 K for 4 hours.

***General method I for shaping the multicomponent catalyst and catalytic assay***

In a general experimental method, the reaction is carried out in a 316L stainless steel fixed-bed reactor, having an inner diameter of 7.8 mm, equipped with a 600 W heating resistor wound on the outside thereof, controlled by a PID controller, and a type K thermocouple covered by a 316L stainless steel sheath and inserted axially in the centre of the catalyst bed.

For the preparation of the catalyst bed, the two components of the catalyst were separately shaped into microparticles and microparticles in the size range of 200 - 400 $\mu$m were isolated by sieving. In turn, silicon carbide microparticles (SiC, Fisher Chemical, mean granule size about 696 $\mu$m) in the size range of 600 - 800 $\mu$m were isolated. Predetermined masses of the microparticles of the two components of the multicomponent catalyst were mixed uniformly, and the resulting mixture was mixed in turn with SiC microparticles until making a total volume of 1.8 ml, which was introduced into the fixed-bed tubular reactor on a quartz wool support.

Before the catalytic experiment, the multicomponent catalyst was subjected to an in situ activation treatment, in other words, in the tubular reactor itself. Said activation treatment consisted of heating from room temperature to 773 K (with a temperature ramp of 3 K/min) under N$_2$ flow (Abelló-Linde, 99.999%) at approximately 50 ml/min, followed by an isothermal step at 773 K for 4 hours and cooling to room temperature. Next, the catalytic conversion experiment was started. To that end, a gas stream flow containing DME/CO/H$_2$/Ar in molar ratios of 1/45/45/9 fed from a pressurised cylinder (Abelló-Linde) was established in the reactor and the reactor was pressurised to the desired reaction pressure through a diaphragm pressure regulating valve (Swagelok) located downstream of the reactor. Next, the stream flow of DME/CO/H$_2$/Ar was adjusted to obtain the desired space velocity (GHSV) and the reactor temperature was increased to the desired reaction temperature following a heating ramp of 3 K/min. The outlet stream of the tubular reactor was depressurised at the pressure control valve and directed to an online Agilent 7890 gas chromatograph equipped with two analysis channels. A first channel equipped with a HayeSep R 80/100 (6ft) packed column, an HP-PLOT-Q 30m (20$\mu$m film thickness) capillary column and an HP-PLOT 5A 30m (12$\mu$m film thickness) molecular sieve capillary column and two TCD detectors for the analysis of permanent gases, and a second analysis channel equipped with a DB 1-MS (60 m) capillary column and an FID detector for the analysis of hydrocarbon and oxygenated compounds.

### General method II of shaping the multicomponent catalyst and catalytic assay

In another general experimental method, an experimental procedure was followed as indicated in the general method I, except in the step of preparing the catalyst bed. According to this general method II, the two components of the multicomponent catalyst were separately shaped into microparticles and microparticles in the size range of 200 - 400 $\mu$m were isolated by sieving. In turn, silicon carbide (SiC) microparticles in the size range of 600 - 800 $\mu$m were isolated. Predetermined masses of the microparticles of each of the two components of the multicomponent catalyst were mixed, separately, with SiC microparticles until a uniform mixture was achieved. Subsequently, the mixture of the microparticles of the ketonisation component with SiC was introduced into the fixed-bed tubular reactor on a quartz wool support. Quartz wool was then added as a divider (3 mm) and the mixture of the microparticles of the carbonylation component with SiC was added into the fixed-bed reactor, being located upstream of the bed formed by the particles of the ketonisation component in the direction of gas flow in the reactor. The total volume of the catalyst bed was 1.8-3.2 ml.

### General method III of shaping the multicomponent catalyst and catalytic assay

In another general experimental method, an experimental procedure was followed as indicated in the general method I, except in the step of preparing the catalyst bed. According to this general method III, the two components of the multicomponent catalyst were mixed in the form of fine powders with the help of a ceramic mortar until a powdered solid with a uniform appearance was obtained. Next, this solid multicomponent was shaped into microparticles and microparticles in the size range of 200 - 400 $\mu$m were isolated by sieving. In turn, silicon carbide (SiC) microparticles in the size range of 600 - 800 $\mu$m were isolated. A predetermined mass of the microparticles of the multicomponent catalyst were mixed with SiC microparticles until making a total volume of 1.8 ml, which was introduced into the fixed-bed tubular reactor on a quartz wool support.

### General method IV of shaping the multicomponent catalyst and catalytic assay

In another general experimental method, an experimental procedure was followed as indicated in the general method I, except in the step of preparing the catalyst bed and in the in situ activation procedure and subsequent catalytic conversion experiment. According to this general method IV, the components of the multicomponent catalyst were separately shaped into microparticles and microparticles in the size range of 200 - 400 $\mu$m were isolated by sieving. In turn, silicon carbide (SiC) microparticles in the size range of 600 - 800 $\mu$m were isolated. Predetermined masses of the microparticles of each of the two components of the multicomponent catalyst were mixed, separately, with SiC microparticles until a uniform mixture was achieved. Subsequently, the mixture of the microparticles of the ketonisation component with SiC was introduced into the fixed-bed tubular reactor on a quartz wool support. Quartz wool was then added as a divider (3 mm) and the mixture of the microparticles of the carbonylation component with SiC was added to the fixed-bed reactor, being located upstream of the particles of the ketonisation component in the direction of gas flow in the reactor. Then, quartz wool was added as a divider (3 mm) and the mixture of the microparticles of the hydrogenation component with SiC was added to the fixed-bed reactor, being located upstream of the particles of the carbonylation component in the direction of gas flow in the reactor. The total volume of the catalyst bed was 1.8 ml.

Before the catalytic experiment, the multicomponent catalyst was subjected to an in situ activation treatment, in other words, in the tubular reactor itself. Said activation treatment consisted of heating from room temperature to 773 K (with a temperature ramp of 3 K/min) under $N_2$ flow (Abelló-Linde, 99.999%) at approximately 50 ml/min, followed by an isothermal step at 773 K for 4 hours and cooling to room temperature. In addition, the multicomponent catalyst was subjected to a second in situ activation step consisting of heating from room temperature to 523 K (with a temperature ramp of 3 K/min) under $H_2$ flow (Abelló-Linde, 99.999%) at approximately 20 ml/min, diluted in $N_2$ (Abelló-Linde, 99.999%), at 50 ml $N_2$/min, followed by an isothermal step at 523 K for 3 hours and cooling to room temperature. Next, the catalytic conversion experiment was started. To that end, a gas stream flow containing CO/$H_2$/Ar in molar ratios of 45/45/10 fed from a pressurised cylinder (Abelló-Linde) was established in the reactor and the reactor was pressurised to the desired reaction pressure through a diaphragm pressure regulating valve (Swagelok) located downstream of the reactor. Next, the stream flow of CO/$H_2$/Ar was adjusted to obtain the desired space velocity (GHSV) and the reactor temperature was increased to the desired reaction temperature following a heating ramp of 3 K/min. The outlet stream of the tubular reactor was depressurised at the pressure control valve and directed to an online Agilent 7890 gas chromatograph equipped with two analysis channels, as described in the general method I.

***General method V of shaping the multicomponent catalyst and catalytic assay***

In another general experimental method, an experimental procedure was followed as indicated in the general method I, except in the step of preparing the catalyst bed. According to this general method V, a single component of the multicomponent catalyst was shaped into microparticles in the size range of 200-400 $\mu$m by sieving. In turn, silicon carbide (SiC) microparticles in the size range of 600 - 800 $\mu$m were isolated. A predetermined mass of the component microparticles of the multicomponent catalyst were mixed with SiC microparticles until a uniform mixture was achieved. Subsequently, the mixture of the component microparticles of the multicomponent catalyst with SiC was introduced into the fixed-bed tubular reactor on a quartz wool support. The total volume of the catalyst bed was 1.8 ml.

**Example 1:**

In an example according to the present invention, the multicomponent catalyst contained 209 mg of H-ETL zeolite as a carbonylation component and 452 mg of $CeO_2$-$ZrO_2$ (Sigma Aldrich, 99.0%) as a ketonisation component. The formation of the multicomponent catalyst and the catalytic assay were carried out according to the general method II.

**Example 2:**

In an example according to the present invention, the multicomponent catalyst contained 158 mg of H-ETL zeolite as a carbonylation component and 347 mg of $CeO_2$-$ZrO_2$ (Sigma Aldrich, 99.0%) as a ketonisation component. The formation of the multicomponent catalyst and the catalytic assay were carried out according to the general method III.

**Example 3**

In an example according to the present invention, the multicomponent catalyst contained 180 mg of H-ETL zeolite as a carbonylation component and 396 mg of $CeO_2$-$ZrO_2$ (Sigma Aldrich, 99.0%) as a ketonisation component. The formation of the multicomponent catalyst and the catalytic assay were carried out according to the general method I.

**Example 4:**

In an example according to the present invention, the multicomponent catalyst contained 125 mg of Ag-H-MOR zeolite as a carbonylation component and 419 mg of $CeO_2$-$ZrO_2$ (Sigma Aldrich, 99.0%) as a ketonisation component. The formation of the multicomponent catalyst and the catalytic assay were carried out according to the general method II.

**Example 5:**

In an example according to the present invention, the multicomponent catalyst contained 202 mg of H-ETL zeolite as a carbonylation component and 452 mg of $Pr_2O_3$/$Al_2O_3$ as a ketonisation component. The formation of the multicomponent catalyst and the catalytic assay were carried out according to the general method II.

**Example 6:**

In an example according to the present invention, the multicomponent catalyst contained 198 mg of Ag-H-MOR zeolite as a carbonylation component and 424 mg of Pr-$CeO_2$ as a ketonisation component. The formation of the multicomponent catalyst and the catalytic assay were carried out according to the general method II.

**Example 7:**

In an example according to the present invention, the multicomponent catalyst contained 150 mg of Ag-H-MOR zeolite as a carbonylation component and 525 mg of $Pr_2O_3$/$Al_2O_3$ as a ketonisation component. The formation of the multicomponent catalyst and the catalytic assay were carried out according to the general method II.

**Example 8:**

In an example according to the present invention, the multicomponent catalyst contained 700 mg of mod-Ag-H-MOR zeolite as a carbonylation component and 1.80 g of H-FER/Pd-CeO$_2$-ZrO$_2$ as a ketonisation component. The formation of the multicomponent catalyst and the catalytic assay were carried out according to the general method II, with the exception that the activation treatment was carried out at a temperature of 598 K.

**Example 9:**

In an example according to the present invention, the multicomponent catalyst contained 198 mg of CuO/ZnO/Al$_2$O$_3$ and 103 mg of γ-Al$_2$O$_3$ as a hydrogenation component, 106 mg of Ag-H-MOR zeolite as a carbonylation component and 503 mg of CeO$_2$-ZrO$_2$ as a ketonisation component. The formation of the multicomponent catalyst and the catalytic assay were carried out according to the general method IV.

**Example 10:**

In an example according to the present invention, the multicomponent catalyst contained 197 mg of Cu/Zn/Al$_2$O$_3$ and 102 mg of γ-Al$_2$O$_3$ as a hydrogenation component, 107 mg of Ag-H-MOR zeolite as a carbonylation component and 499 mg of CeO$_2$-ZrO$_2$ as a ketonisation component. The formation of the multicomponent catalyst and the catalytic assay were carried out according to the general method IV.

**Example 11:**

In an example according to the present invention, the multicomponent catalyst contained 199 mg of CuO/ZnO/Al$_2$O$_3$ and 105 mg of γ-Al$_2$O$_3$ as a hydrogenation component, 106 mg of Ag-H-MOR zeolite as a carbonylation component and 496 mg of CeO$_2$-ZrO$_2$ as a ketonisation component. The formation of the multicomponent catalyst and the catalytic assay were carried out according to the general method IV.

**Example 12:**

In a comparative example, not according to the present invention, the multicomponent catalyst contained 183 mg of Ag-H-MOR zeolite as a carbonylation component and 403 mg of WOs (Sigma Aldrich, 99.9%) as a ketonisation component. The formation of the multicomponent catalyst and the catalytic assay were carried out according to the general method II.

**Example 13:**

In a comparative example, not according to the present invention, the multicomponent catalyst contained 184 mg of H-BEA zeolite (SiO$_2$/Al$_2$O$_3$ = 27, TosoH) as a carbonylation component and 407 mg of CeO$_2$-ZrO$_2$ (Sigma Aldrich, 99.0%) as a ketonisation component. The formation of the multicomponent catalyst and the catalytic assay were carried out according to the general method II.

**Example 14:**

In a comparative example, not according to the present invention, the catalyst contained only one carbonylation component, in other words, 500 mg of H-MOR zeolite. The formation of the catalyst and the catalytic assay were carried out according to the general method V.

**Example 15:**

In a comparative example, not according to the present invention, the catalyst contained only one carbonylation component, in other words, 399 mg of H-ETL zeolite. The formation of the catalyst and the catalytic assay were carried out according to the general method V.

**Example 16:**

In a comparative example, not according to the present invention, the catalyst contained only one ketonisation component, in other words, 350 mg of CeO$_2$-ZrO$_2$. The formation of the catalyst and the catalytic assay were carried out according to the general method V.

Table 1. *Acetone productivity and selectivities in the conversion of synthesis gas (examples 9-11) and mixtures of synthesis gas and DME (examples 1-8) according to illustrative examples of the present invention (examples 1-11) and comparative examples not according to the present invention (examples 12-16) determined after 50-1000 minutes of reaction in flow.*

| Example | $T^{(1)}$ (K) | GHSV[2] ($h^{-1}$) | $r^{(3)}$ ($g_{acetone}$ $kg_{cat}^{-1}$ $h^{-1}$) | Selectivity[4] (C%) | | | |
|---|---|---|---|---|---|---|---|
| | | | | $CH_4$ | AcOOMe[5] | Acetone | Others[6] |
| Example 1 | 548 | 1867 | 6.1 | 16.9 | 4.6 | 74.5 | 4.0 |
| Example 2 | 548 | 2418 | 3.0 | 27.0 | 1.9 | 60.2 | 11.0 |
| Example 3 | 548 | 2157 | 3.6 | 26.5 | 1.6 | 60.1 | 11.8 |
| Example 4 | 548 | 2489 | 20.6 | 1.8 | 27.7 | 33.4 | 37.1 |
| Example 5 | 548 | 1244 | 2.7 | 35.5 | <0.2 | 55.2 | 9.4 |
| Example 6 | 548 | 2240 | 18.8 | 1.7 | 23.8 | 25.7 | 48.8 |
| Example 7 | 548 | 1400 | 11.8 | 1.2 | 49.0 | 18.2 | 31.6 |
| Example 8 | 548 | 431 | 5.6 | 3.2 | 4.0 | 72.3 | 20.5 |
| Example 9 | 523 | 1445 | 2.4 | 3.8 | <0.2 | 46.3 | 49.9 |
| Example 10 | 548 | 1387 | 8.3 | 2.9 | 4.5 | 45.0 | 47.6 |
| Example 11 | 573 | 1445 | 19.4 | 3.8 | 6.0 | 30.8 | 59.4 |
| Example 12 | 548 | 3446 | 0.2 | 1.1 | 62.4 | 0.2 | 36.4 |
| Example 13 | 548 | 4073 | <0.2 | 0.5 | <0.2 | <0.2 | 99.5 |
| Example 14 | 523 | 3086 | <0.2 | 4.8 | 42.4 | <0.2 | 52.8 |
| Example 15 | 548 | 3068 | <0.2 | 8.0 | 88.6 | <0.2 | 3.4 |
| Example 16 | 548 | 6222 | <0.2 | 3.4 | <0.2 | <0.2 | 96.6 |

(1) Reaction temperature.
(2) GHSV: Gas Hourly Space Velocity.
(3) *r* = acetone productivity:

$$r = [w_{acetone}]/[M_{cat}]$$

where $w_{acetone}$ is the mass flow of acetone produced and $M_{cat}$ is the mass of the multifunctional catalyst.
(4) Selectivity between organic products on a methanol-free basis. Given the fact that methanol can be recirculated as a reagent for the process, alternatively after a step of dehydration to DME, it is reasonable to express selectivities to organic compounds on a methanol-free basis. In other words, the selectivity to each organic reaction product "i" ($S_i$) is calculated with the following equation:

$$S_i=[N_{i,outlet}*C_i]/[\Sigma N_{j,outlet}*C_j]*100$$

where $N_{i,outlet}$ is the molar flow of the product "i" in the reactor outlet stream (mol $h^{-1}$), and the sum in the denominator extends to all organic products "j" except methanol.
(5) AcOOMe: methyl acetate.
(6) Others: Other organic compounds: DME, $C_{2+}$ hydrocarbons, and other oxygenated compounds such as acetic acid.

As can be deduced from the results presented in Table 1, examples 1-11 according to the present invention give rise to a process where selectivities to acetone are obtained that are much higher than those obtained in the comparative examples 12-16, not according to the present invention, in which selectivity to acetone is low to essentially zero. In turn, the examples show that the presence of at least one carbonylation component and one ketonisation component in the multicomponent catalyst is essential for the production of acetone with high selectivity.

Although the present invention has been described in terms of preferred embodiments, it is understood that said description should not be interpreted as limiting the invention described herein. After reading the description, it will be immediately apparent to those with common knowledge in the field, in view of the teachings of this invention, that several alterations and modifications may be made thereto. The attached claims are to be interpreted as encompassing all such alterations and modifications that fall within the spirit and scope of the present invention.

**Claims**

1.  A process for the direct synthesis of acetone comprising, at least, the following steps:

    a) reacting a feed stream comprising, at least, synthesis gas on a solid multicomponent catalyst; wherein said multicomponent catalyst integrates at least one carbonylation active component and one ketonisation active component; wherein said carbonylation component comprises a zeotype material having a network structure comprising 8-membered ring units; wherein said ketonisation component comprises a hydroxide, oxide or any combination thereof selected from the list of yttrium, zirconium, titanium, aluminium, silicon, vanadium, niobium, tantalum, chromium, molybdenum, manganese, zinc, gallium, indium, tin, bismuth, lanthanide elements, or any combination thereof;
    b) recovering acetone from the stream of said reaction step.

2.  The process according to claim 1, wherein the zeotype material is a zeolite selected from the group consisting of MOR, ETL, FER, CHA, SZR structures or any combination thereof.

3.  The process according to claim 2, wherein the zeolite incorporates at least one trivalent element selected from Al, Ga, B, In, Y, La, Fe or any combination thereof.

4.  The process according to claim 3, wherein the trivalent element is Al and the zeolite has a $SiO_2/Al_2O_3$ molar ratio comprised between 3 and 100.

5.  The process according to any of the preceding claims, wherein the zeolite comprises a metal deposited on the surface thereof, selected from silver, copper, palladium, iridium, platinum, rhodium, rhenium, zinc, and any combination thereof.

6.  The process according to claim 5, wherein the zeolite has been modified by adding a metal selected from silver, copper, palladium, and any combination thereof.

7.  The process according to claim 1, wherein the ketonisation component of the multicomponent catalyst comprises $CeO_2$, $ZrO_2$ or any combination thereof.

8.  The process according to claim 7, wherein the ketonisation component of the multicomponent catalyst further comprises elements selected from the list of manganese, titanium, other lanthanides, and any combination thereof.

9.  The process according to any of the preceding claims, wherein the multicomponent catalyst comprises, in turn, a hydrogenation component.

10. The process according to claim 9, wherein the hydrogenation component of the multicomponent catalyst comprises an oxide selected from ZnO, $ZrO_2$, MgO, $In_2O_3$, $Ga_2O_3$, $CeO_2$ or any combination thereof.

11. The process according to any of claims 9 or 10, wherein the hydrogenation component of the multicomponent catalyst comprises oxide-supported copper selected from ZnO, $ZrO_2$, MgO, $In_2O_3$, $Ga_2O_3$, $CeO_2$ or any combination thereof.

12. The process according to any of claims 9 to 11, wherein the hydrogenation component of the multicomponent catalyst further comprises an acidic solid selected from $Al_2O_3$, zeolite or any combination thereof.

13. The process according to any of the preceding claims, wherein the multicomponent catalyst is formed as a composite material from the individual components in their powder form.

14. The process according to any of the preceding claims, wherein the multicomponent catalyst comprises a mixture of shaped bodies of the individual components.

15. The process according to any of the preceding claims, wherein the feed stream further comprises organic compounds selected from methanol, dimethyl ether, or any combination thereof.

16. The process according to any of the preceding claims, wherein the reaction is carried out in a single reactor.

17. The process according to any of the preceding claims, wherein the reaction temperature is in the range of 373 K to 673 K.

18. The process according to any of the preceding claims, wherein the reaction pressure is in the range of 1 bar to 200 bar.

19. The process according to any of the preceding claims, wherein the $H_2$/CO molar ratio in the feed stream is between 0.1 and 4.

20. The process according to any of the preceding claims, wherein the $CO_2$/CO molar ratio in the feed stream is in the range of 0 to 2.

21. The process according to any of the preceding claims, wherein a stream comprising carbon monoxide, carbon dioxide, hydrogen, methanol, DME, acetic acid, methyl acetate, or any combination thereof, is recovered from the reactor effluent stream and recirculated to the reactor.

22. The process according to claim 21, wherein the stream comprising methanol recovered from the reactor effluent stream is subjected to a dehydration step, in another reactor, where methanol is converted, fully or partially, into DME, followed by a step of total or partial removal of water, prior to its recirculation to the reactor of the process of the invention.

**Figure 1**

**Figure 2**

# INTERNATIONAL SEARCH REPORT

| International application No. |
| --- |
| PCT/ES2021/070931 |

## A. CLASSIFICATION OF SUBJECT MATTER

*C07C49/08* (2006.01)
*C07C45/49* (2006.01)
According to International Patent Classification (IPC) or to both national classification and IPC

## B. FIELDS SEARCHED

Minimum documentation searched (classification system followed by classification symbols)
C07C

Documentation searched other than minimum documentation to the extent that such documents are included in the fields searched

Electronic data base consulted during the international search (name of data base and, where practicable, search terms used)

EPODOC, WPI, INVENES

## C. DOCUMENTS CONSIDERED TO BE RELEVANT

| Category* | Citation of document, with indication, where appropriate, of the relevant passages | Relevant to claim No. |
| --- | --- | --- |
| A | CN 111517929 A (DALIAN INST CHEM & PHYSICS CAS) 11/08/2020, paragraphs [0025 - 0035] | 1-22 |
| A | CN 104193606 A (SHANXI COAL CHEM INST ET AL.) 10/12/2014, paragraphs [0004 - 0008] | 1-22 |
| A | GB 2212494 A (BP CHEM INT LTD) 26/07/1989, page 2, line 3 - page 4, line 8 | 1-22 |

☐ Further documents are listed in the continuation of Box C.    ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means. | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | | |
| | | "&" | document member of the same patent family |

| Date of the actual completion of the international search | Date of mailing of the international search report |
| --- | --- |
| 17/03/2022 | **(18/03/2022)** |
| Name and mailing address of the ISA/ | Authorized officer |
| OFICINA ESPAÑOLA DE PATENTES Y MARCAS<br>Paseo de la Castellana, 75 - 28071 Madrid (España)<br>Facsimile No.: 91 349 53 04 | J. Peces Aguado<br><br>Telephone No. 91 3496870 |

Form PCT/ISA/210 (second sheet) (January 2015)

## INTERNATIONAL SEARCH REPORT

Information on patent family members

International application No.

PCT/ES2021/070931

| Patent document cited in the search report | Publication date | Patent family member(s) | Publication date |
|---|---|---|---|
| CN111517929 A | 11.08.2020 | NONE | |
| CN104193606 A | 10.12.2014 | NONE | |
| GB2212494 A | 26.07.1989 | NONE | |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9295978 B2, A. Schäfer
- US 8669295 B2, Q. Fu
- CN 102653777 B

- CN 111517929 A
- CN 104193606 A
- GB 2212494 A

**Non-patent literature cited in the description**

- **J. P. LANGE.** Methanol synthesis: a short review of technology improvements. *Catalysis Today,* 2001, vol. 64 (1-2), 3-8
- **H. BATENI ; C. ABLE.** Development of Heterogeneous Catalysts for Dehydration of Methanol to Dimethyl Ether: A Review. *Catalysis in Industry,* 2019, vol. 11, 7-33

- **V. BERZIN et al.** Selective production of acetone during continuous synthesis gas fermentation by engineered biocatalyst Clostridium sp. MAceT113. *Letters in Applied Microbiology,* 2012, vol. 55, 149-154
- Handbook of Heterogeneous Catalysis. Wiley-VCH Verlag GmbH & Co, 2008, vol. 1
- Synthesis of solid catalysts. Wiley-VCH Verlag GmbH & Co, 2009